# EUROPÄISCHE PATENTANMELDUNG

(11) **EP 3 644 086 A1**
(43) Veröffentlichungstag der Anmeldung: **29.04.2020**
(21) Anmeldenummer: 18202461.2
(22) Anmeldetag: 25.10.2018
(51) Int. Cl.: G01R 33/561, G01R 33/56, A61B 5/055

(54) **MAGNETRESONANZ-FINGERPRINTING-VERFAHREN FÜR AUFNAHMEN MIT KONTRASTMITTEL**

(71) Anmelder: Bayer AG, 51373 Leverkusen (DE)
(72) Erfinder: Die Erfindernennung liegt noch nicht vor
(74) Vertreter: BIP Patents

(57) **Zusammenfassung**

Die vorliegende Erfindung befasst sich mit dem technischen Gebiet der Magnetresonanztomografie. Gegenstände der vorliegenden Erfindung sind ein Verfahren, ein System und ein Computerprogrammprodukt zur Ermittlung verschiedener Zustände eines Kontrastmittels in verschiedenen Gewebearten in einem Magnetresonanz-Fingerprinting-Verfahren.

## Beschreibung

Die vorliegende Erfindung befasst sich mit dem technischen Gebiet der Magnetresonanztomografie. Gegenstände der vorliegenden Erfindung sind ein Verfahren, ein System und ein Computerprogrammprodukt zur Ermittlung verschiedener Zustände eines Kontrastmittels in verschiedenen Gewebearten in einem Magnetresonanz-Fingerprinting-Verfahren.

Die Magnetresonanztomographie, abgekürzt MRT oder MR (engl. MRI: *Magnetic Resonance Imaging*)*,* ist ein bildgebendes Verfahren, das vor allem in der medizinischen Diagnostik zur Darstellung von Struktur und Funktion der Gewebe und Organe im menschlichen oder tierischen Körper eingesetzt wird.

Bei der MR-Bildgebung werden die magnetischen Momente von Protonen in einem Untersuchungsobjekt in einem Grundmagnetfeld ausgerichtet, sodass sich eine makroskopische Magnetisierung entlang einer Längsrichtung einstellt. Diese wird anschließend durch das Einstrahlen von Hochfrequenz(HF)-Pulsen aus der Ruhelage ausgelenkt (Anregung). Die Rückkehr der angeregten Zustände in die Ruhelage (Relaxation) bzw. die Magnetisierungsdynamik wird anschließend mittels einer oder mehrerer HF-Empfangsspulen als Relaxationssignale detektiert.

Zur Ortskodierung werden dem Grundmagnetfeld schnell geschaltete magnetische Gradientenfelder überlagert. Die erfassten Relaxationssignale bzw. die detektierten und ortsaufgelösten MR-Daten liegen zunächst als Rohdaten in einem Ortsfrequenzraum vor, und können durch anschließende Fourier-Transformation in den Ortsraum (Bildraum) transformiert werden.

Bei der nativen MRT werden die Gewebs-Kontraste durch die unterschiedlichen Relaxationszeiten (T1 und T2) und die Protonendichte erzeugt.

Die T1-Relaxation beschreibt den Übergang der Längsmagnetisierung (longitudinale Magnetisierung) in ihren Gleichgewichtszustand, wobei T1 diejenige Zeit ist, die benötigt wird, um 63,21% der Gleichgewichtsmagnetisierung vor der Resonanzanregung zu erreichen. Sie wird auch longitudinale Relaxaktionszeit oder Spin-Gitter-Relaxationszeit genannt.

Die T2-Relaxation beschreibt in analoger Weise den Übergang der Transversalmagnetisierung in ihren Gleichgewichtszustand.

MR-Kontrastmittel entfalten ihre Wirkung, indem sie die Relaxationszeiten der Strukturen, die Kontrastmittel aufnehmen, verändern. Es lassen sich zwei Stoffgruppen unterscheiden: para- und superparamagnetische Stoffe. Beide Stoffgruppen besitzen ungepaarte Elektronen, die ein magnetisches Feld um die einzelnen Atome bzw. Moleküle induzieren.

Superparamagnetische führen zu einer überwiegenden T2-Verkürzung, während paramagnetische Kontrastmittel im Wesentlichen zu einer T1-Verkürzung führen.

Die Wirkung dieser Kontrastmittel ist indirekt, da das Kontrastmittel selbst kein Signal abgibt, sondern nur die Signalintensität in seiner Umgebung beeinflusst.

Ein Beispiel für ein superparamagnetisches Kontrastmittel sind Eisenoxidnanopartikel (SPIO, engl.: *superparamagnetic iron oxide*)*.*

Beispiele für paramagnetische Kontrastmittel sind Gadolinium-Chelate wie Gadopentetat-Dimeglumin (Handelsname: Magnevist® u.a.), Gadotersäure (Dotarem®, Dotagita®, Cyclolux®), Gadodiamid (Omniscan®), Gadoteridol (ProHance®) und Gadobutrol (Gadovist®).

Kontrastmittel auf der Basis der Gadoxetsäure zeichnen sich dadurch aus, dass sie spezifisch von Leberzellen, den Hepatozyten, aufgenommen werden, sich im Funktionsgewebe (Parenchym) anreichern und die Kontraste in gesundem Lebergewebe verstärken. Die Zellen von Zysten, Metastasen und der meisten Leberzellkarzinome arbeiten nicht mehr wie normale Leberzellen, nehmen das Kontrastmittel nicht oder kaum auf, werden nicht verstärkt dargestellt und werden damit erkennbar und lokalisierbar. Beispiele für Kontrastmittel auf der Basis der Gadoxetsäure sind in US 6,039,931A beschrieben; sie sind kommerziell zum Beispiel unter den Markennamen Primovist® oder Eovist® erhältlich.

Der kontrastverstärkende Effekt von Primovist®/Eovist® wird durch den stabilen Gadoliniumkomplex Gd-EOB-DTPA (Gadolinium-Ethoxybenzyl-Diethylentriamin-Pentaessigsäure) vermittelt. DTPA bildet mit dem paramagnetischen Gadoliniumion einen Komplex, der eine extrem hohe thermodynamische Stabilität aufweist. Der Ethoxybenzylrest (EOB) ist der Vermittler der hepatobiliären Aufnahme des Kontrastmittels. Dabei wird die hepatozelluläre Aufnahme durch ein organisches Anion-Transportsystem vermittelt, welches auch an der Aufnahme von Bilirubin in die Leberzelle beteiligt ist (O. Clement et al., Gadolinium-ethoxybenzyl-DTPA, a new liver-specific magnetic resonance contrast agent. Kinetic and enhancementpatterns in normal and cholestatic rats; Invest Radiol (1992); 27: 612-619).

Mittels Magnetresonanz-Fingerprinting-Verfahren lassen sich detailliertere Aufnahmen von einem Untersuchungsobjekt in kürzerer Zeit erzeugen. Magnetresonanz-Fingerprinting-Verfahren sind beispielsweise beschrieben in Ma et al., Magnetic Resonance Fingerprinting, Nature, 495: 187-192 (2013); Jiang et al., MR Fingerprinting Using Fast Imaging with Steady State Precession (FISP) with Spiral Readout, Magnetic Resonance in Medicine 74: 1621-1631 (2015) oder Cloos et al., Online Radial Multiband Magnetic Resonance Fingerprinting, ISMRM 2016: 608.

Ein Magnetresonanz-Fingerprinting-Verfahren ist ein quantitatives Messverfahren, bei dem zunächst mehrere Magnetresonanz-Bilder eines Untersuchungsobjekts bei variierenden Aufnahmeparametern erzeugt werden. Die Aufnahmeparameter werden dabei üblicherweise in einer pseudorandomisierten Weise variiert. Für die einzelnen Bildpunkte (Voxel) der Magnetresonanz-Bilder wird dann ein ortsabhängiger Magnetresonanz-Signalverlauf generiert. Der ermittelte Signalverlauf wird anschließend für jedes Voxel mit mehreren in einer Datenbank hinterlegten Signalverläufen verglichen, wobei jedem der Signalverläufe in der Datenbank ein spezifischer Wert mindestens eines Gewebeparameters zugeordnet ist. Die Datenbank-Signalverläufe stellen zu erwartende Signalverläufe dar und werden vorab ermittelt und/oder berechnet. Ein Volumenelement einer Probe, bei dem der Wert des mindestens einen Gewebeparameters dem spezifischen Datenbankwert entspricht, sollte in einer Magnetresonanz-Messung den in der Datenbank gespeicherten Signalverlauf zeigen. Wird also ein übereinstimmender Signalverlauf identifiziert, so lässt sich aus der Datenbank der dazugehörige Wert des mindestens einen Gewebeparameters auslesen.

Auf diese Weise kann aus MR-Messdaten die räumliche Verteilung von gewebespezifischen Parametern (wie die Transversalrelaxation T2 oder die Longitudinalrelaxation T1; sogenannte Tl-und T2-Karten) in dem abgebildeten Untersuchungsobjekt quantitativ ermittelt werden. Ein Vorteil der Magnetresonanz-Fingerprinting-Methode ist dabei, dass mehrere Gewebeparameter gleichzeitig in einer einzelnen Messung akquiriert werden können.

C. E. Anderson et al. berichten über die gleichzeitige Messung von Gewebeparametern von zwei gleichzeitig verabreichten Kontrastmitteln (Dual Contrast - Magnetic Resonance Fingerprinting (DCMRF): A Platform for Simultaneous Quantification of Multiple MRI Contrast Agents; Scietntific Reports 7; 8431: 1-10; DOI:10.1038/s41598-017-08762-9). Das Verfahren nutzt die Tatsache, dass sich die verwendeten Kontrastmittel in ihren Relaxivitäten unterscheiden und sich daher differenzieren lassen.

Wird ein Kontrastmittel verabreicht, so verteilt es sich im Körper. Eine gemessene Relaxationsänderung wird durch das Kontrastmittel, das sich in verschiedenen Gewebsbereichen befinden kann, verursacht. Die Signale können dabei von verschiedenen Gewebearten stammen (z.B. Funktionsgewebe (Parenchym), Zwischengewebe (Interstitium) und/oder einer Gewebsflüssigkeit (z.B. Blut, Lymphe, Galle).

Gesucht wird nach einer Lösung, mit der verschiedene Gewebsarten, die in einem Volumenelement vorhanden sind, in einer einzigen Messung mit nur einem Kontrastmittel differenziert werden können.

Diese Aufgabe wird durch die Gegenstände der unabhängigen Patentansprüche gelöst. Bevorzugte Ausführungsformen finden sich in den abhängigen Patentansprüchen sowie in der vorliegenden Beschreibung und den Figuren.

Ein erster Gegenstand der vorliegenden Erfindung ist ein Verfahren umfassend die folgenden Schritte:
- Bereitstellen einer Magnetresonanz-Fingerprint-Datenbank,
   - wobei die Magnetresonanz-Fingerprint-Datenbank Datenbank-Signalverläufe für ein spezifisches Kontrastmittel in mindestens zwei verschiedenen Gewebearten, einer ersten Gewebeart und einer zweiten Gewebeart, umfasst,
   - wobei das Kontrastmittel in der ersten Gewebeart einen anderen Zustand aufweist, als in der zweiten Gewebeart,
- Erfassen eines Magnetresonanz-Signalverlaufs für ein Volumenelement eines Untersuchungsbereichs mittels einer Magnetresonanz-Fingerprinting-Methode unter Verwendung des Kontrastmittels,
- Vergleichen des Magnetresonanz-Signalverlaufs mit Datenbank-Signalverläufen,
- Identifizieren eines Datenbank-Signalverlaufs mit einer definierten Übereinstimmung mit dem Magnetresonanz-Signalverlauf,
- Ermitteln der Zustände des Kontrastmittels in dem Volumenelement,
- Ausgeben von Informationen zu den Zuständen des Kontrastmittels in dem Volumenelement.

Ein weiterer Gegenstand der vorliegenden Erfindung ist ein System umfassend
- eine Empfangseinheit,
- eine Steuereinheit,
- eine Signalvergleichseinheit und
- eine Ausgabeneinheit,
   - wobei die Steuereinheit konfiguriert ist, die Empfangseinheit zu veranlassen, für mindestens ein Volumenelement eines Untersuchungsbereichs einen Magnetresonanz-Signalverlauf zu empfangen, wobei der Magnetresonanz-Signalverlauf in einem Magnetresonanz-Fingerprinting-Verfahren unter Verwendung eines Kontrastmittels generiert worden ist,
   - wobei die Steuereinheit konfiguriert ist, die Empfangseinheit zu veranlassen, mehrere Datenbank-Signalverläufe aus einer Magnetresonanz-Fingerprint-Datenbank zu empfangen, wobei jedem Signalverlauf mindestens zwei Datenbankwerte von zwei Gewebeparametern zugeordnet sind, einem ersten Gewebeparameter und einem zweiten Gewebeparameter, wobei der erste Gewebeparameter einen ersten Zustand des Kontrastmittels in einer ersten Gewebeart charakterisiert und der zweite Gewebeparameter einen zweiten Zustand des Kontrastmittels in einer zweiten Gewebeart charakterisiert,
   - wobei die Steuereinheit konfiguriert ist, die Signalvergleichseinheit zu veranlassen, den Magnetresonanz-Signalverlauf mit den Datenbank-Signalverläufen zu vergleichen, einen Datenbank-Signalverlauf mit einer definierten Übereinstimmung zu identifizieren und die zu dem identifizierten Datenbank-Signalverlauf zugeordneten mindestens zwei Datenbankwerte zu ermitteln,
   - wobei die Steuereinheit konfiguriert ist, die Ausgabeeinheit zu veranlassen, die mindestens zwei Datenbankwerte oder aus den mindestens zwei Datenbankwerten abgeleitete Werte abzuspeichern und/oder auszugeben.

Ein weiterer Gegenstand der vorliegenden Erfindung ist ein Computerprogrammprodukt umfassend ein Computerprogramm, das in einen Arbeitsspeicher eines Computers geladen werden kann und dort den Computer dazu veranlasst, folgende Schritte ausführen:
- Empfangen eines Magnetresonanz-Signalverlaufs für ein Volumenelement eines Untersuchungsbereichs aus einem Magnetresonanz-Fingerprinting-Verfahren unter Verwendung eines Kontrastmittels,
- Empfangen von Datenbank-Signalverläufen, wobei jedem Datenbank-Signalverlauf verschiedene Zustände des Kontrastmittels in verschiedenen Gewebearten zugeordnet sind,
- Vergleichen des Magnetresonanz-Signalverlaufs mit Datenbank-Signalverläufen,
- Identifizieren eines Datenbank-Signalverlaufs mit einer definierten Übereinstimmung mit dem Magnetresonanz-Signalverlauf,
- Ermitteln der verschiedenen Zustände des Kontrastmittels in dem Volumenelement,
- Ausgeben von Informationen zu den verschiedenen Zuständen des Kontrastmittels in dem Volumenelement.

Ein weiterer Gegenstand ist die Verwendung eines Kontrastmittels in einem Magnetresonanz-Fingerprinting-Verfahren, wobei das Kontrastmittel in mindestens zwei verschiedenen Gewebearten, unterschiedliche Zustände aufweist, zur Bestimmung der unterschiedlichen Zustände in einem abgebildeten Volumenelement.

Ein weiterer Gegenstand ist ein Kontrastmittel zur Verwendung in einem Magnetresonanz-Fingerprinting-Verfahren, wobei das Kontrastmittel in mindestens zwei verschiedenen Gewebearten unterschiedliche Zustände aufweist, wobei die unterschiedlichen Zustände in einem abgebildeten Volumenelement in einer Messung gleichzeitig erfasst werden.

Die Erfindung wird nachstehend näher erläutert, ohne zwischen den Erfindungsgegenständen (Verfahren, System, Computerprogrammprodukt, Verwendung, Kontrastmittel zur Verwendung) zu unterscheiden. Die nachfolgenden Erläuterungen sollen vielmehr für alle Erfindungsgegenstände in analoger Weise gelten, unabhängig davon, in welchem Kontext (Verfahren, System, Computerprogrammprodukt, Verwendung, Kontrastmittel zur Verwendung) sie erfolgen.

Wenn in der vorliegenden Beschreibung oder in den Patentansprüchen Schritte in einer Reihenfolge genannt sind, bedeutet dies nicht zwingend, dass die Erfindung auf die genannte Reihenfolge beschränkt ist. Vielmehr ist denkbar, dass die Schritte auch in einer anderen Reihenfolge oder auch parallel zueinander ausgeführt werden; es sei denn, ein Schritt baut auf einem anderen Schritt auf, was zwingend erforderlich macht, dass der aufbauende Schritt nachfolgend ausgeführt wird (was im Einzelfall aber deutlich wird). Die genannten Reihenfolgen stellen damit bevorzugte Ausführungsformen der Erfindung dar.

Die vorliegende Erfindung nutzt ein Magnetresonanz-Fingerprinting-Verfahren, um verschiedene Zustände eines Kontrastmittels in unterschiedlichen Gewebearten in einer Messung gleichzeitig zu erfassen.

Unter einer Gewebeart wird eine Ansammlung differenzierter Zellen einschließlich ihrer extrazellulären Matrix verstanden. Beispiele für Gewebearten sind Epithelgewebe, Binde- und Stützgewebe (Knochengewebe, Knorpelgewebe, Fettgewebe), Muskel-Gewebe, Nervengewebe und Lebergewebe. Vorliegend werden auch Gewebsflüssigkeiten bzw. flüssige Gewebe (z.B. Blut, Lymphe, Galle) zu den Gewebearten gezählt. Ferner können krankhaftes und gesundes Gewebe zwei unterschiedliche Gewebearten darstellen.

Unter einem Kontrastmittel wird ein Stoff oder Stoffgemisch verstanden, dessen Anwesenheit in einer Magnetresonanzmessung zu einem veränderten Signal führt. Vorzugsweise führt das Kontrastmittel zu einer Verkürzung der T1-Zeitspanne und/oder der T2-Zeitspanne.

Das Kontrastmittel liegt in mindestens zwei Gewebearten in unterschiedlichen Zuständen vor. Das bedeutet, dass es eine erste Gewebeart und eine zweite Gewebeart gibt. Kontrastmittel, das einem Untersuchungsobjekt verabreicht wird, verteilt sich in den Gewebearten; verabreichtes Kontrastmittel liegt demnach sowohl in der ersten als auch in der zweiten Gewebeart vor. Wie sich das Kontrastmittel auf die Gewebearten verteilt, lässt sich prinzipiell experimentell ermitteln und beispielsweise durch einen Verteilungskoeffizienten angeben. Ein solcher Verteilungskoeffizient kann beispielsweise der Quotient aus der Konzentration des Kontrastmittels in der ersten Gewebeart und der Konzentration des Kontrastmittels in der zweiten Gewebeart sein. Es ist denkbar, dass der Verteilungskoeffizient selbst konzentrationsabhängig ist, das heißt, dass er sich mit der Konzentration des Kontrastmittels in einer Gewebeart ändern kann.

Das Kontrastmittel weist in der ersten Gewebeart einen anderen Zustand auf als in der zweiten Gewebeart. Ein anderer Zustand führt zu einer veränderten Relaxation. Ein Zustand kann durch mindestens einen Wert eines Gewebeparameters charakterisiert werden. In einer Ausführungsform bedeuten die Begriffe "anderer Zustand" oder "unterschiedliche (verschiedene) Zustände", dass das Kontrastmittel in der ersten Gewebeart eine andere molare T1-Relaxivität aufweist als in der zweiten Gewebeart. In einer anderen Ausführungsform bedeuten die Begriffe "anderer Zustand" oder "unterschiedliche (verschiedene) Zustände", dass das Kontrastmittel in der ersten Gewebeart eine andere molare T2-Relaxivität aufweist als in der zweiten Gewebeart. In einer weiteren Ausführungsform bedeuten die Begriffe "anderer Zustand" oder "unterschiedliche (verschiedene) Zustände", dass das Kontrastmittel in der ersten Gewebeart sowohl eine andere molare T1-Relaxivität als auch eine andere molare T2-Relaxivität aufweist als in der zweiten Gewebeart.

Die molare Relaxivität ist ein gebräuchliches Maß für die Wirksamkeit, das heißt für die relaxationsverkürzende Wirkung eines Kontrastmittels. Ihre Einheit kann in (s·Mol/L)⁻¹ angegeben werden (Sekunde x Mol / Liter)⁻¹.

Vorzugsweise ist die molare Relaxivität in der ersten Gewebeart mindestens um den Faktor 2 größer als in der zweiten Gewebeart; besonders bevorzugt ist sie mindestens um den Faktor 2,5 größer. In einer besonders bevorzugten Ausführungsform unterscheiden sich die molaren Relaxivitäten in den zwei verschiedenen Gewebearten um einen Faktor von mindestens 2 (vorzugsweise mindestens 2,5) bei einer Magnetfeldstärke des Grundmagnetfeldes von mindestens 1,5 Tesla, vorzugsweise bei mindestens 2 Tesla, noch mehr bevorzugt bei mindestens 2,5 Tesla, noch mehr bevorzugt bei mindestens 3 Tesla, am meisten bevorzugt bei einer Magnetfeldstärke von 0,4 Tesla bis mindestens 3 Tesla.

In einer besonders bevorzugten Ausführungsform handelt es sich bei dem Kontrastmittel um einen Stoff oder ein Stoffgemisch mit der Gadoxetsäure oder einem Salz der Gadoxetsäure als kontrastverstärkenden Wirkstoff. Ganz besonders bevorzugt handelt es sich um das Dinatriumsalz der Gadoxetsäure (Gd-EOB-DTPA Dinatrium). Gd-EOB-DTPA weist in Leberzellen (Hepatozyten) eine signifikant höhere molare Relaxivität auf als beispielsweise in Plasma.

Tabelle 1 zeigt die molaren Relaxivitäten (in (s·mMol/L)⁻¹) von Gd-EOB-DTPA in verschiedenen Gewebearten bei unterschiedlichen Magnetfeldstärken des Grundmagnetfeldes.

**Tab. 1**

| | 0,47 T | 1,14 T | 1,5 T | 3 T |
|---|---|---|---|---|
| Wasser | 5,3⁽¹⁾ | | 4,7⁽¹⁾ | 4,3⁽¹⁾ |
| Galle | | 5,6 ± 0,3 | 5,6 ± 0,4 | 5,6 ± 0,3 |
| Urin | | 5,5 ± 0,1 | 5,5 ± 0,2 | 5,6 ± 0,1 |
| Plasma | 8,7⁽¹⁾ | | 6,9⁽¹⁾ | 6,2⁽¹⁾ |
| Blut | 11,0⁽²⁾ | 8,1 ± 0,2 | 7,3⁽¹⁾/8,3 ± 0,3 | 7,2 ± 0,1 |
| Lebergewebe | 16,6⁽²⁾ | 14,0 ± 2,8 | 16,4 ± 3,1 | 13,4 ± 4,0 |

| | | | | |
|---|---|---|---|---|
| (1): Rohrer et al. Radiol 2005; 40(11): 715-724 (2): Schuhmann-Giampieri et al. Radiol. 1992; 183: 59-64 | | | | |

Überraschend ist, dass die hohe molare Relaxivität in Lebergewebe auch bei hohen (klinisch relevanten) Magnetfeldstärken erhalten bleibt.

Die Erfindung ermöglicht es, Kontrastmittel, das sich in der ersten Gewebeart befindet, von Kontrastmittel, das sich in der zweiten Gewebeart befindet, zu unterscheiden, selbst wenn sich beide Gewebearten in demselben Volumenelement befinden, das in Magnetresonanz-Bilddaten als ein Voxel abgebildet wird.

Das Untersuchungsobjekt ist üblicherweise ein Lebewesen, vorzugsweise ein Säugetier, ganz besonders bevorzugt ein Mensch. Der Untersuchungsbereich ist ein Teil des Untersuchungsobjekts, zum Beispiel ein Organ wie die Leber oder ein Teil eines Organs.

Der Untersuchungsbereich, auch Aufnahmevolumen (engl.: *field of view,* FOV) genannt, stellt insbesondere ein Volumen dar, welches in den aufgenommenen Magnetresonanz-Bilddaten abgebildet wird. Der Untersuchungsbereich wird typischerweise durch einen Nutzer, beispielsweise auf einer Übersichtsaufnahme (engl.: *localizer*) festgelegt. Selbstverständlich kann der Untersuchungsbereich alternativ oder zusätzlich auch automatisch, beispielsweise auf Grundlage eines ausgewählten Protokolls, festgelegt werden.

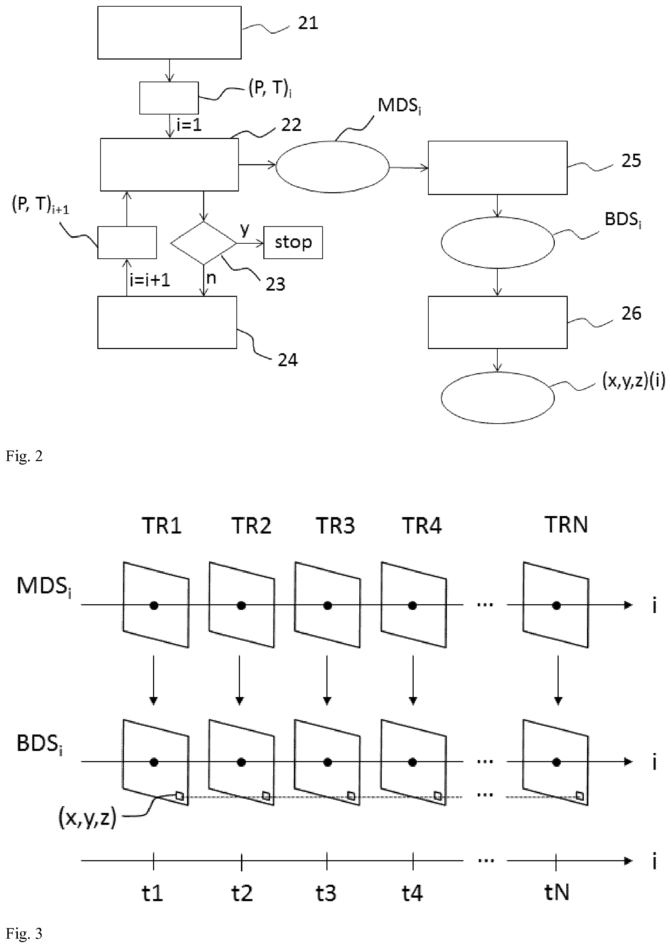

Dem Untersuchungsobjekt wird ein Kontrastmittel verabreicht, das sich in dem Untersuchungsbereich verteilt. Der Untersuchungsbereich wird in ein Grundmagnetfeld eingebracht. Der Untersuchungsbereich wird einem Magnetresonanz-Fingerprinting-Verfahren unterzogen und es wird für mindestens ein Volumenelement des Untersuchungsbereichs ein Magnetresonanz-Signalverlauf ermittelt (Details hierzu siehe Fig. 2 und die dazu gehörige Beschreibung).

Die Magnetresonanz-Fingerprinting-Methode sieht typischerweise ein Erfassen eines Magnetresonanz-Signalverlaufs für eine Vielzahl von Voxeln mittels eines pseudorandominisierten bzw. inkohärenten Aufnahmeschemas vor. Es ist auch denkbar, dass der Magnetresonanz-Signalverlauf aus einem Bereich erfasst wird, welcher gröber aufgelöst als ein Voxel ist. Dann kann der Magnetresonanz-Signalverlauf beispielsweise gemittelt über mehrere Voxel erfasst werden.

Die Magnetresonanz-Fingerprinting-Methode umfasst insbesondere, dass für die Aufnahme der verschiedenen Magnetresonanz-Signale verschiedene Aufnahmeparameter gesetzt werden. Die Aufnahmeparameter können dabei in einer pseudorandominisierten bzw. inkohärenten Weise variiert werden. Mögliche Aufnahmeparameter, welche bei der Akquisition des Magnetresonanz-Signalverlaufs verändert werden, sind beispielsweise eine Echozeit, eine Ausbildung und/oder Anzahl von Hochfrequenz-Pulsen, eine Ausbildung und/oder Anzahl von Gradientenpulsen, eine Diffusionskodierung usw. Auf diese Weise kann mittels der Magnetresonanz-Fingerprinting-Methode ein für das Voxel charakteristischer Magnetresonanz-Signalverlauf, ein sogenannter Fingerabdruck (Fingerprint) des Voxels, erfasst werden.

In einem weiteren Schritt des erfindungsgemäßen Verfahrens werden die Magnetresonanz-Signalverläufe mit Signalverläufen, die in einer Magnetresonanz-Fingerprint-Datenbank gespeichert sind, verglichen (Datenbank-Signalverläufe).

Die Datenbank-Signalverläufe können beispielsweise in einer Kalibrierungsmessung ermittelt werden und/oder simuliert werden. Verfahren zur Erzeugung von Datenbank-Signalverläufen sind im Stand der Technik beschrieben (siehe zum Beispiel J. Xie et al.: Fast dictionary generation and searching for magnetic resonance fingerprinting, Conf Proc IEEE Eng Med Biol Soc. 2017 Jul;2017:3256-3259. doi: 10.1109/EMBC.2017.8037551).

Die Magnetresonanz-Fingerprinting-Methode sieht typischerweise vor, dass ein Datenbank-Signalverlauf dem erfassten Magnetresonanz-Signalverlauf anhand des Ergebnisses des Signalvergleichs zugeordnet wird. Der Signalvergleich kann dabei eine Bestimmung einer Ähnlichkeit des erfassten Magnetresonanz-Signalverlaufs mit den mehreren Datenbank-Signalverläufen umfassen, wobei derjenige Datenbank-Signalverlauf dem Magnetresonanz-Signalverlauf zugeordnet wird, welcher die größte Ähnlichkeit mit dem Magnetresonanz-Signalverlauf aufweist.

Es ist auch denkbar, dass ein Ähnlichkeitsschwellenwert festgelegt wird, der erreicht werden muss, um eine Zuordnung vorzunehmen.

Verfahren zum Vergleich und zur Identifizierung von Ähnlichkeiten sind im Stand der Technik beschrieben (siehe z.B. S.F. Cauley et al., Fast group matching for MR fingerprinting reconstruction, Magnetic Resonance in Medicine 74:523-528 (2015)).

Den verschiedenen Datenbank-Signalverläufen sind jeweils spezifische Datenbankwerte von Gewebeparametern zugeordnet.

Die zu dem zugeordneten Datenbank-Signalverlauf gehörenden Datenbankwerte können dann beispielsweise als Messwerte von Gewebeparametern des betrachteten Volumenelements gesetzt werden. Als Ergebnis des Signalvergleichs können zum Beispiel die mittels der Magnetresonanz-Fingerprinting-Methode gewonnenen Messwerte der Gewebeparameter für das entsprechende Voxel ausgegeben bzw. abgespeichert werden.

Eine Auswahl möglicher Gewebeparameter, welche mittels der Magnetresonanz-Fingerprinting Methode quantifiziert werden können, ist: eine T1-Relaxationszeit, eine T2-Relaxationszeit, ein Diffusionswert (beispielsweise ein scheinbarer Diffusionskoeffizient, *apparent diffusion coefficient,* ADC), ein Magnetisierungsmoment, eine Protonendichte, eine Resonanzfrequenz, eine Konzentration eines Stoffs, usw. Selbstverständlich sind auch weitere, dem Fachmann als sinnvoll erscheinende Gewebeparameter denkbar.

Erfindungsgemäß stehen die Datenbankwerte bzw. Messwerte der Gewebeparameter im Zusammenhang mit den verschiedenen Zuständen des verwendeten Kontrastmittels in den mindestens zwei verschiedenen Gewebearten.

Wenn in einem betrachteten Volumenelement zwei verschiedene Gewebearten vorhanden sind und nach Verabreichung eines Kontrastmittels ein Magnetresonanz-Signalverlauf für das Volumenelement erfasst wird, können durch die Identifizierung des zugehörigen Datenbank-Signalverlaufs beispielsweise die T1-Relaxationszeit des Kontrastmittels in der einen Gewebeart und die T1-Relaxationszeit in der anderen Gewebeart gleichzeitig als zugehörige Datenbankwerte ermittelt und daraus beispielsweise die Konzentration des Kontrastmittels in der ersten Gewebeart und die Konzentrationszeit des Kontrastmittels in der zweiten Gewebeart für das entsprechende Volumenelement errechnet werden.

Es können die ermittelten Datenbankwerte und/oder aus den Datenbankwerten abgeleitete Werte abgespeichert und/oder ausgegeben werden. Ein abgeleiteter Wert ist üblicherweise ein Wert, der sich durch eine Berechnung aus dem Datenbankwert ergibt. Ein Beispiel für einen abgeleiteten Wert ist die Konzentration des Kontrastmittels in einer Gewebeart.

Ein Gegenstand der vorliegenden Erfindung ist ein System, mit dem das erfindungsgemäße Verfahren durchgeführt werden kann.

Das System umfasst eine Empfangseinheit, eine Steuereinheit, eine Signalvergleichseinheit und eine Ausgabeeinheit. Es ist denkbar, dass die genannten Einheiten Bestandteile eines einzigen Computersystems sind; es ist aber auch denkbar, dass die genannten Einheiten Bestandteile von mehreren separaten Computersystemen sind, die über ein Netzwerk miteinander verbunden sind, um Daten und/oder Steuersignale von einer Einheit zu einer anderen Einheit zu übermitteln.

Ein "Computersystem" ist ein System zur elektronischen Datenverarbeitung, das mittels programmierbarer Rechenvorschriften Daten verarbeitet. Ein solches System umfasst üblicherweise einen "Computer", diejenige Einheit, die einen Prozessor zur Durchführung logischer Operationen umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabegeräte dienen. Beispiele hierfür sind Monitor (Bildschirm), Drucker, Scanner, Maus, Tastatur, Laufwerke, Kamera, Mikrofon, Lautsprecher etc. Auch interne Anschlüsse und Erweiterungskarten gelten in der Computertechnik als Peripherie.

Computersysteme von heute werden häufig in Desktop-PCs, Portable PCs, Laptops, Notebooks, Netbooks und Tablet-PCs und so genannte Handhelds (z.B. Smartphone) unterteilt; alle diese Systeme können zur Ausführung der Erfindung genutzt werden.

Die Eingaben in das Computersystem erfolgen über Eingabemittel wie beispielsweise eine Tastatur, eine Maus, ein Mikrofon und/oder dergleichen. Als Eingabe soll auch die Auswahl eines Eintrags aus einem virtuellen Menü oder einer virtuellen Liste oder das Anklicken eines Auswahlkästchens und dergleichen verstanden werden.

Das erfindungsgemäße System ist konfiguriert, mindestens einen Magnetresonanz-Signalverlauf und mehrere Datenbank-Signalverläufe zu empfangen, den mindestens einen Magnetresonanz-Signalverlauf mit den Datenbank-Signalverläufen zu vergleichen, als Ergebnis des Vergleichs einen Datenbank-Signalverlauf mit einer definierten Übereinstimmung mit dem Magnetresonanz-Signalverlauf zu identifizieren, mindestens zwei Datenbankwerte, die dem identifizierten Datenbank-Signalverlauf zugeordnet sind, zu ermitteln, und die ermittelten Datenbankwerte oder von den ermittelten Datenbankwerten abgeleitete Werte abzuspeichern und/oder auszugeben.

Die Steuereinheit dient der Steuerung der Empfangseinheit, der Signalvergleichseinheit und der Ausgabeeinheit und der Koordinierung der Daten- und Signalflüsse zwischen verschiedenen Einheiten. Es ist denkbar, dass mehrere Steuereinheiten vorhanden sind.

Die Empfangseinheit dient zum Empfang des mindestens einen Magnetresonanz-Signalverlaufs. Der mindestens eine Magnetresonanz-Signalverlauf kann beispielsweise von einer Magnetresonanzanlage übermittelt werden oder aus einem Datenspeicher ausgelesen werden. Die Magnetresonanzanlage kann ein Bestandteil des erfindungsgemäßen Systems sein. Denkbar ist aber auch, dass das erfindungsgemäße System ein Bestandteil einer Magnetresonanzanlage ist.

Die Empfangseinheit dient ferner zum Empfang von mehreren Datenbank-Signalverläufen aus einer Magnetresonanz-Fingerprinting-Datenbank. Die Magnetresonanz-Fingerprinting-Datenbank kann ein Bestandteil des erfindungsgemäßen Systems sein. In der Magnetresonanz-Fingerprinting-Datenbank ist eine Vielzahl an Datenbank-Signalverläufen gespeichert. Jedem Datenbank-Signalverlauf sind spezifische Werte (Datenbankwerte) von Gewebeparametern zugeordnet. Die Datenbank-Signalverläufe geben den zu erwartenden Signalverlauf einer Probe an, die entsprechende Werte der Gewebeparameter aufweist. Ein Teil der Datenbank-Signalverläufe gibt die zu erwartenden Signalverläufe einer Probe an, bei der in einem Volumenelement mindestens zwei verschiedene Gewebearten vorhanden sind und ein verabreichtes Kontrastmittel in den mindestens zwei Gewebearten verschiedene Zustände aufweist. Der eine Zustand ist durch einen spezifischen Wert (Datenbankwert) eines ersten Gewebeparameters charakterisiert, der andere Zustand durch einen spezifischen Wert (Datenbankwert) eines zweiten Gewebeparameters.

Von der Empfangseinheit werden der mindestens eine Magnetresonanz-Signalverlauf und die mehreren Datenbank-Signalverläufe an die Signalvergleichseinheit übermittelt.

Die Signalvergleichseinheit ist konfiguriert, den mindestens einen Magnetresonanz-Signalverlauf mit verschiedenen Datenbank-Signalverläufen zu vergleichen, um einen Datenbank-Signalverlauf zu identifizieren, der eine definierte Übereinstimmung mit dem Magnetresonanz-Signalverlauf aufweist. Ist der Datenbank-Signalverlauf identifiziert, werden die dem Datenbank-Signalverlauf zugeordneten Datenbankwerte ermittelt.

Die Ausgabeeinheit ist konfiguriert, die ermittelten Datenbankwerte abzuspeichern und/oder auszugeben. Eine Speicherung erfolgt üblicherweise in einem Datenspeicher, der Bestandteil des erfindungsgemäßen Systems ist oder mit diesem über einem Netzwerk verbunden ist. Eine Ausgabe erfolgt üblicherweise auf einem Bildschirm. Es ist denkbar, dass die Ausgabe in Form eines zwei- oder dreidimensionalen Bildes erfolgt. Es ist denkbar, dass das Bild den betrachteten Untersuchungsbereich darstellt und für einzelne Volumenelemente eine Farbe und/oder ein Grauton auf Basis eines oder mehrerer für das Volumenelement ermittelter Datenbankwerte ausgewählt ist.

Es ist denkbar, dass auf Basis der ermittelten Datenbankwerte abgeleitete Werte berechnet werden. Die Berechnung kann beispielsweise durch die Steuereinheit und/oder die Signalvergleichseinheit erfolgen.

Die Erfindung wird nachstehend anhand von Figuren näher erläutert, ohne die Erfindung auf die in den Figuren gezeigten Merkmale oder Merkmalskombinationen beschränken zu wollen.

Es zeigen:

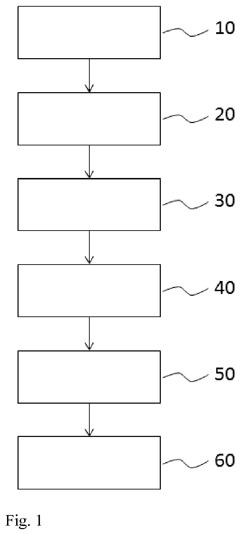

Figur 1 zeigt schematisch einen Ablauf des erfindungsgemäßen Verfahrens. Die Abfolge umfasst die Schritte
   - Bereitstellen einer Magnetresonanz-Fingerprint-Datenbank (10)
   - Erfassen eines Magnetresonanz-Signalverlaufs für ein Volumenelement eines Untersuchungsbereichs mittels einer Magnetresonanz-Fingerprinting-Methode unter Verwendung eines Kontrastmittels (20)
   - Vergleichen des Magnetresonanz-Signalverlaufs mit Datenbank-Signalverläufen (30)
   - Identifizieren eines Datenbank-Signalverlaufs mit einer definierten Übereinstimmung mit dem Magnetresonanz-Signalverlauf (40)
   - Ermitteln der Zustände des Kontrastmittels in dem Volumenelement (50)
   - Ausgeben von Informationen zu den Zuständen des Kontrastmittels in dem Volumenelement (60).
Figur 2 zeigt schematisch eine bevorzugte Ausführungsform für das Erfassen eines Magnetresonanz-Signalverlaufs mittels einer Magnetresonanz-Fingerprinting-Methode (Schritt 20 in Figur 1).

Es wird eine Pulssequenz z.B. entsprechend einem gewünschten Kontrast oder anderer gewünschter Eigenschaften der mit der Pulssequenz auslesbaren Messdaten auf übliche Weise gewählt (21).

Die Pulssequenz wird mit einem ersten Satz (i=1) an Aufnahmeparametern Pᵢ durchgeführt, wobei Messdaten entlang einer ersten k-Raum-Trajektorie Tᵢ abgetastet werden sollen (P, T)ᵢ. Eine k-Raum-Trajektorie entlang derer Messdaten in einer Wiederholung gemessen werden, kann den k-Raum kartesisch, spiralförmig, radial oder auch in einer Kombination der vorgenannten Abtastarten oder entlang einer frei erdachten Trajektorie abtasten.

Gemäß der Pulssequenz werden HF-Pulse in den Untersuchungsbereich eingestrahlt, Gradienten geschaltet und die durch die eingestrahlten HF-Pulse und die geschalteten Gradienten erzeugten Echosignale ausgelesen (22). Dabei werden nach einer Anregung mit einem HF-Anregungspuls Messdaten entlang der k-Raum-Trajektorie Tᵢ aufgenommen und in einem Messdatensatz MDS; gespeichert.

Aus jedem Messdatensatz MDSᵢ wird ein Bilddatensatz BDSᵢ rekonstruiert (25), wobei auch nur ein Teil der in dem Messdatensatz MDSᵢ enthaltenen Messdaten für die Rekonstruktion verwendet werden kann. Somit erhält man einen Bilddatensatz BDSᵢ pro Wiederholung i, d.h. insgesamt N Bilddatensätze BDSᵢ.

In einer Abfrage (23) wird abgefragt, ob bereits alle N gewünschten Wiederholungen durchgeführt und die entsprechenden N Messdatensätze MSDᵢ gespeichert wurden. Ist dies nicht der Fall ("n"), werden eine k-Raum-Trajektorie für die nächste Wiederholung gewählt und die Parameter der Pulssequenz entsprechend angepasst und ggf. zusätzlich variiert (24). In der Regel wird sich eine gewählte weitere k-Raum-Trajektorie Tᵢ₊₁ von einer vorhergehenden k-Raum-Trajektorie Tᵢ unterscheiden.

Mit dem so erhaltenen nächsten Parametern Pᵢ₊₁ und der gewählten weiteren k-Raum-Trajektorie Tᵢ₊₁ ((P, T)ᵢ₊₁) wird die Pulssequenz wiederholt und somit eine erneute Messung (22) durchgeführt, derart, dass in aufeinanderfolgenden Wiederholungen Messdaten entlang den gewählten k-Raum-Trajektorien Tᵢ, Tᵢ₊₁ gemessen werden. Zur Wahl von Trajektorien sei auf die umfangreiche Literatur zu Magnetresonanz-Fingerprinting-Verfahren verwiesen; als Beispiel sei die Offenlegungsschrift DE102016217675A1 genannt, deren Inhalt durch Bezugnahme vollständig in diese Beschreibung aufgenommen wird.

Wurden bereits alle N gewünschten Wiederholungen durchgeführt und die entsprechenden N Messdatensätze MSDᵢ gespeichert ("y"), wird keine weitere Messung durchgeführt ("stop"), und es wird für mindestens ein Voxel (x,y,z) in den rekonstruierten Bilddatensätzen BDSᵢ eine Voxel-Zeit-Serie (x,y,z)(i) gebildet, die eine Signalintensität des Voxels (x,y,z) im Verlauf der Aufnahmezeiten (und damit im Verlauf der nacheinander durchgeführten Wiederholungen (i) der Messdatensätze MDSᵢ) wiedergibt. Üblicherweise wird eine solche Voxel-Zeit-Serie (x,y,z)(i) für alle Voxel (x,y,z), die in dem interessierenden Untersuchungsbereich liegen, durchgeführt. Die gebildeten Voxel-Zeit-Serien (x,y,z)(i) werden gespeichert.

Figur 3 zeigt schematisch den Zusammenhang von Messdatensätzen MDSᵢ und Bilddatensätzen BDSᵢ im zeitlichen Verlauf, d.h. im Verlauf von i.

In der obersten Zeile sind die Messdatensätze MDSi, wie sie nacheinander in den Wiederholungen TRi aufgenommen wurden, dargestellt, wobei beispielhaft die Wiederholungen i = 1, i = 2, i = 3, i = 4 und i = N explizit dargestellt sind. In der zweiten Zeile sind die aus den Messdatensätzen MDSi rekonstruierten Bilddatensätze BDSi in gleicher Weise dargestellt, wobei beispielhaft ein Voxel (x,y,z) in den Bilddatensätzen BDSi markiert ist. Beispielsweise zu diesem Voxel (x,y,z) kann die jeweilige Intensität des Voxels (x,y,z) zu den, den Wiederholungen Ti entsprechenden Zeiten Ti gegen die Zeit als Voxel-Zeit-Serie aufgetragen werden. Eine Voxel-Zeit-Serie (x,y,z)(i) ist ein Magnetresonanz-Signalverlauf für ein Volumenelement des Untersuchungsbereichs.

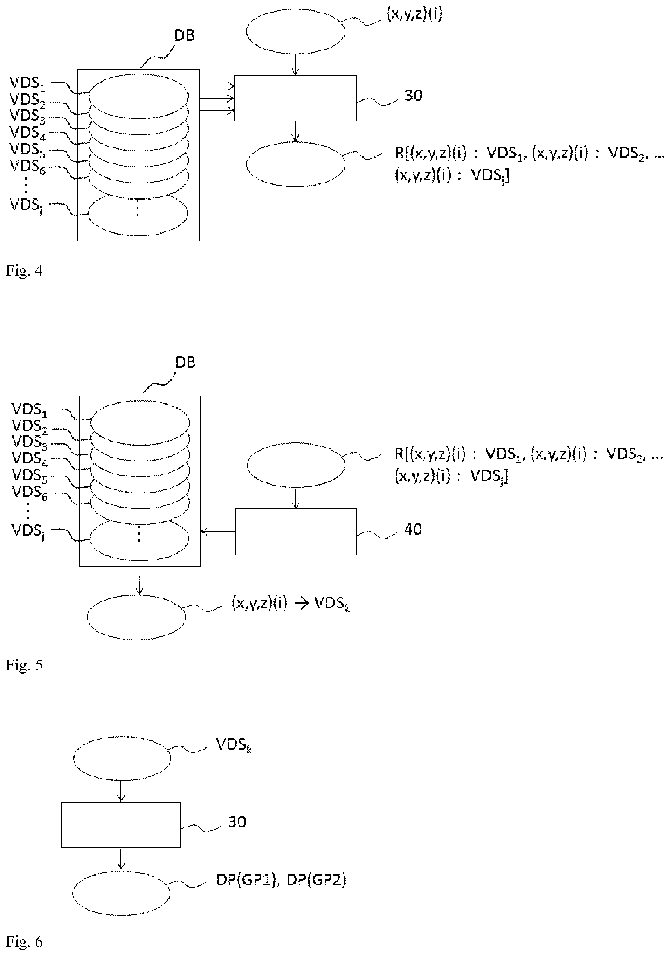

Figur 4 zeigt schematisch den Vergleich von Magnetresonanz-Signalverläufen mit Datenbank-Signalverläufen (Schritt 30 in Figur 1).

Jede gespeicherte Voxel-Zeit-Serie (x,y,z)(i) aus Figur 2 wird mit mehreren der Datenbank-Signalverläufe VDSₗ bis VDSⱼ, die in einer Magnetresonanz-Fingerprinting-Datenbank (DB) gespeichert sind, verglichen (30). Das Ergebnis eines jeden Vergleichs kann ein Ähnlichkeitswert R sein, der angibt, wie ähnlich eine Voxel-Zeit-Serie (x,y,z)(i) einem Datenbank-Signalverlauf ist.

Figur 5 zeigt schematisch die Identifizierung eines Datenbank-Signalverlaufs mit einer definierten Übereinstimmung mit dem Magnetresonanz-Signalverlauf (Schritt 40 in Figur 1).

Üblicherweise wird jeder Voxel-Zeit-Serie (x,y,z)(i) ein Datenbank-Signaverlauf VDSₖ zugeordnet, üblicherweise derjenige Datenbank-Signaverlauf, der die größte Übereinstimmung mit der Voxel-Zeit-Serie (x,y,z)(i) aufweist (maximaler R-Wert).

Figur 6 zeigt schematisch die Ermittlung der Zustände des Kontrastmittels für das Volumenelement, das in der jeweiligen Voxel-Zeit-Serie (x,y,z)(i) abgebildet ist (Schritt 50 in Figur 1). Die Zustände des Kontrastmittels werden durch spezifische Werte von zwei Gewebeparametern GP1 und GP2 charakterisiert. Diese Werte (Datenbankwerte) ergeben sich aus dem in Schritt 50 identifizierten Datenbank-Signaverlauf VDSₖ. Sie sind üblicherweise in der Magnetresonanz-Fingerprinting-Datenbank zum Datenbank-Signaverlauf VDSₖ gespeichert und können nach Identifizierung von VDSₖ ausgelesen werden.

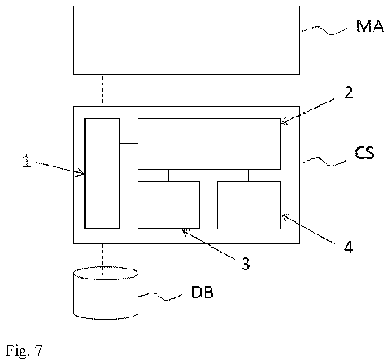

Figur 7 zeigt schematisch eine Ausführungsform des erfindungsgemäßen Systems. Das System umfasst eine Empfangseinheit (1), eine Steuereinheit (2), eine Signalvergleichseinheit (3) und eine Ausgabeeinheit (4). Die genannten Einheiten sind Bestandteile eines Computersystems (CS). Die Empfangseinheit (1) ist konfiguriert, Magnetresonanz-Signalverläufe von einer Magnetresonanzanlage (MA) und Datenbank-Signalverläufe von einer Magnetresonanz-Fingerprinting-Datenbank (DB) zu empfangen. Der Empfang kann über ein Netzwerk (dargestellt durch gestrichelte Linien) erfolgen.

## Patentansprüche

1. Verfahren umfassend die folgenden Schritte:
• Bereitstellen einer Magnetresonanz-Fingerprint-Datenbank,
- wobei die Magnetresonanz-Fingerprint-Datenbank Datenbank-Signalverläufe für ein spezifisches Kontrastmittel in mindestens zwei verschiedenen Gewebearten, einer ersten Gewebeart und einer zweiten Gewebeart, umfasst,
- wobei das Kontrastmittel in der ersten Gewebeart einen anderen Zustand aufweist, als in der zweiten Gewebeart,
• Erfassen eines Magnetresonanz-Signalverlaufs für ein Volumenelement eines Untersuchungsbereichs mittels einer Magnetresonanz-Fingerprinting-Methode unter Verwendung des Kontrastmittels,
• Vergleichen des Magnetresonanz-Signalverlaufs mit Datenbank-Signalverläufen,
• Identifizieren eines Datenbank-Signalverlaufs mit einer definierten Übereinstimmung mit dem Magnetresonanz-Signalverlauf,
• Ermitteln der Zustände des Kontrastmittels in dem Volumenelement,
• Ausgeben von Informationen zu den Zuständen des Kontrastmittels in dem Volumenelement.

2. Verfahren gemäß Anspruch 1, wobei das Kontrastmittel in der ersten Gewebeart eine andere molare Relaxivität aufweist als in der zweiten Gewebeart.

3. Verfahren gemäß Anspruch 2, wobei die molare Relaxivität in der ersten Gewebeart mindestens um den Faktor 2 größer, vorzugsweise mindestens um den Faktor 2,5 größer ist als in der zweiten Gewebeart.

4. Verfahren gemäß Anspruch 3, wobei die molare Relaxivität bei einer Magnetfeldstärke des Grundmagnetfeldes von mindestens 1,5 Tesla, vorzugsweise mindestens 2 Tesla, noch mehr bevorzugt mindestens 2,5 Tesla, noch mehr bevorzugt mindestens 3 Tesla, am meisten bevorzugt bei einer Magnetfeldstärke von 0,4 Tesla bis mindestens 3 Tesla in der ersten Gewebeart mindestens um den Faktor 2 größer, vorzugsweise mindestens um den Faktor 2,5 größer ist als in der zweiten Gewebeart.

5. Verfahren gemäß einem der Ansprüche 1 bis 4, wobei das Kontrastmittel in der ersten Gewebeart eine höhere molare T1-Relaxivität aufweist als in der zweiten Gewebeart.

6. Verfahren gemäß einem der Ansprüche 1 bis 5, wobei das Kontrastmittel Gadoxetsäure oder ein Salz der Gadoxetsäure als kontrastverstärkenden Wirkstoff umfasst.

7. Verfahren gemäß einem der Ansprüche 1 bis 6, wobei das Kontrastmittel Gd-EOB-DTPA Dinatrium umfasst.

8. Verfahren gemäß einem der Ansprüche 1 bis 7, wobei es sich bei der ersten Gewebeart um Hepatozyten handelt.

9. Verfahren gemäß einem der Ansprüche 1 bis 8, wobei es sich bei der ersten Gewebeart um gesundes Lebergewebe und bei der zweiten Gewebeart um krankhaftes Lebergewebe handelt.

10. System umfassend
• eine Empfangseinheit,
• eine Steuereinheit,
• eine Signalvergleichseinheit und
• eine Ausgabeneinheit,
- wobei die Steuereinheit konfiguriert ist, die Empfangseinheit zu veranlassen, für mindestens ein Volumenelement eines Untersuchungsbereichs einen Magnetresonanz-Signalverlauf zu empfangen, wobei der Magnetresonanz-Signalverlauf in einem Magnetresonanz-Fingerprinting-Verfahren unter Verwendung eines Kontrastmittels generiert worden ist,
- wobei die Steuereinheit konfiguriert ist, die Empfangseinheit zu veranlassen, mehrere Datenbank-Signalverläufe aus einer Magnetresonanz-Fingerprint-Datenbank zu empfangen, wobei jedem Signalverlauf mindestens zwei Datenbankwerte von zwei Gewebeparametern zugeordnet sind, einem ersten Gewebeparameter und einem zweiten Gewebeparameter, wobei der erste Gewebeparameter einen ersten Zustand des Kontrastmittels in einer ersten Gewebeart charakterisiert und der zweite Gewebeparameter einen zweiten Zustand des Kontrastmittels in einer zweiten Gewebeart charakterisiert,
- wobei die Steuereinheit konfiguriert ist, die Signalvergleichseinheit zu veranlassen, den Magnetresonanz-Signalverlauf mit den Datenbank-Signalverläufen zu vergleichen, einen Datenbank-Signalverlauf mit einer definierten Übereinstimmung zu identifizieren und die zu dem identifizierten Datenbank-Signalverlauf zugeordneten mindestens zwei Datenbankwerte zu ermitteln,
- wobei die Steuereinheit konfiguriert ist, die Ausgabeeinheit zu veranlassen, die mindestens zwei Datenbankwerte oder aus den mindestens zwei Datenbankwerten abgeleitete Werte abzuspeichern und/oder auszugeben.

11. Computerprogrammprodukt umfassend ein Computerprogramm, das in einen Arbeitsspeicher eines Computers geladen werden kann und dort den Computer dazu veranlasst, folgende Schritte ausführen:
• Empfangen eines Magnetresonanz-Signalverlaufs für ein Volumenelement eines Untersuchungsbereichs aus einem Magnetresonanz-Fingerprinting-Verfahren unter Verwendung eines Kontrastmittels,
• Empfangen von Datenbank-Signalverläufen, wobei jedem Datenbank-Signalverlauf verschiedene Zustände des Kontrastmittels in verschiedenen Gewebearten zugeordnet sind,
• Vergleichen des Magnetresonanz-Signalverlaufs mit Datenbank-Signalverläufen,
• Identifizieren eines Datenbank-Signalverlaufs mit einer definierten Übereinstimmung mit dem Magnetresonanz-Signalverlauf,
• Ermitteln der verschiedenen Zustände des Kontrastmittels in dem Volumenelement,
• Ausgeben von Informationen zu den verschiedenen Zuständen des Kontrastmittels in dem Volumenelement.

12. Verwendung eines Kontrastmittels in einem Magnetresonanz-Fingerprinting-Verfahren, wobei das Kontrastmittel in mindestens zwei verschiedenen Gewebearten, unterschiedliche Zustände aufweist, zur Bestimmung der unterschiedlichen Zustände in einem abgebildeten Volumenelement.

13. Verwendung gemäß Anspruch 12, wobei das Kontrastmittel Gadoxetsäure oder ein Salz der Gadoxetsäure umfasst.

14. Verwendung gemäß Anspruch 12 oder 13, wobei es sich bei der ersten Gewebeart um Hepatozyten handelt.

15. Kontrastmittel zur Verwendung in einem Magnetresonanz-Fingerprinting-Verfahren, wobei das Kontrastmittel in mindestens zwei verschiedenen Gewebearten unterschiedliche Zustände aufweist, wobei die unterschiedlichen Zustände in einem abgebildeten Volumenelement in einer Messung gleichzeitig erfasst werden.

16. Kontrastmittel zur Verwendung gemäß Anspruch 15, wobei das Kontrastmittel Gadoxetsäure oder ein Salz der Gadoxetsäure umfasst.

17. Kontrastmittel zur Verwendung gemäß Anspruch 15 oder 16, wobei es sich bei der ersten Gewebeart um Hepatozyten handelt.
